# EUROPEAN PATENT APPLICATION

(11) **EP 4 850 532 A1**
(43) Date of publication of application: **09.12.2026**
(21) Application number: 25748712.4
(22) Date of filing: 30.01.2025
(51) Int. Cl.: A01N 59/08, A01N 33/12, A01N 39/00, A01P 3/00, A61L 9/01, C02F 1/50, C02F 1/76

(54) **LIQUID COMPOSITION**

(30) Priority: 31.01.2024 JP 2024012398
(71) Applicant: Carlit Co., Ltd., Tokyo 104-0031 (JP); Earth Corporation, Tokyo 101-0048 (JP)
(72) Inventor: NAGAI, Atsushi, Shibukawa-shi, Gunma 377-0004 (JP); NAKAMURA, Manami, Shibukawa-shi, Gunma 377-0004 (JP); YOSHIDA, Yuto, Shibukawa-shi, Gunma 377-0004 (JP); KASHIWAI, Daiki, Tokyo 104-0031 (JP)
(74) Representative: advotec.
(86) International application number: PCT/JP2025/002944
(87) International publication number: WO 2025/164709

(57) **Abstract**

The present invention provides a liquid composition including at least a halous acid species (A), a radical-generating catalyst (B), and a buffering agent (C), the liquid composition being characterized by satisfying prescribed conditions.

## Description

### FIELD

The present invention relates to a liquid composition.

### BACKGROUND

Various disinfectants and microbicides have been developed in order to exhibit a microbicidal effect and a preservative effect. However, among bacteria, there also exist bacteria that form spores, which are cell structures having extremely high resistance, so that general disinfectants (for example, alcohol-based disinfectants) may have insufficient microbicidal effects.

Radicals are important chemical species that are widely used since they are highly reactive and have strong oxidizing power. For example, when bacteria or viruses come into contact with radicals, they are eliminated (in other words, inactivated) by the oxidizing power of the radicals, and hence effects such as microbial reduction and deodorization are obtained. Therefore, radicals are used in various fields such as medical care, hygiene, food processing, and water treatment.

In recent years, highly safe disinfection techniques that use radicals and are applicable also to biological tissues have been developed. For example, PTL 1 discloses an agent comprising a radical generation catalyst and a radical generation source, the radical generation catalyst comprising one or both of: at least one of ammonium and a salt thereof; and a substance having at least one of Lewis acidity and Brønsted acidity.

### [CITATION LIST]

### [PATENT LITERATURE]

[PTL 1] Japanese Unexamined Patent Publication (Kokai) No. 2017-109978

### SUMMARY

### [TECHNICAL PROBLEM]

An object of the present invention is to provide a liquid composition comprising: a radical generation catalyst having excellent long-term stability; a halous acid species; and a buffering agent.

### [SOLUTION TO PROBLEM]

The agent described in PTL 1, which comprises a radical generation catalyst and a radical generation source, particularly a halous acid species, is provided mainly as a liquid composition, is highly reactive, and has strong oxidizing power. In view of this, the present inventors intensively studied in order to provide a high-concentration liquid composition that is capable of maintaining effects (for example, a microbicidal effect) of the agent for a long period, that is suitable for transportation, and that is capable of maintaining the effects even after dilution. As a result, the present inventors discovered that a liquid composition comprising: a radical generation catalyst; a radical generation source (in particular, a halous acid species); and a buffering agent; the liquid composition having excellent long-term stability, can be provided by satisfying certain conditions, thereby completing the present invention. Specifically, the present invention includes the following.
[1] A liquid composition comprising at least:
   a halous acid species (A);
   a radical generation catalyst (B); and
   a buffering agent (C);
   the liquid composition satisfying the following conditions (I) to (IV):
   (I) the halous acid species (A) is at least one selected from the group consisting of a halous acid, a halite ion, and a halite salt, and is contained in the liquid composition at 0.3 to 20.0% by mass;
   (II) the radical generation catalyst (B) is an ammonium salt represented by the following chemical formula (1): 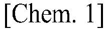 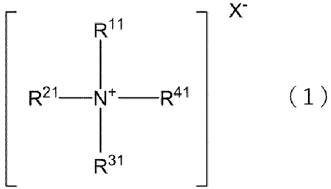 [wherein in the chemical formula (1),
      each of R¹¹, R²¹, R³¹, and R⁴¹ is a hydrogen atom or an alkyl group, and optionally contains an ether bond, a ketone (carbonyl group), an ester bond, or an amide bond, or an aromatic ring; and R¹¹, R²¹, R³¹, and R⁴¹ are the same as or different from each other; or
      two or more of R¹¹, R²¹, R³¹, and R⁴¹ are optionally joined together to form a cyclic structure together with N⁺ to which they are bonded; and the cyclic structure is either saturated or unsaturated, is either an aromatic ring or a non-aromatic ring, and optionally has one or more substituents; and
      X⁻ is an anion (excluding a peroxodisulfate ion)];
      and is contained in the liquid composition at 0.3 to 20.0% by mass;
   (III) the total concentration of the halous acid species (A) and the radical generation catalyst (B) contained in the liquid composition is 1.3 to 30.0% by mass; and
   (IV) the buffering agent (C) contained in the liquid composition contains phosphate ions; the concentration of phosphate ions contained in the liquid composition is 0.01 to 1.50% by mass; and the mass ratio of phosphate ions is not less than 0.33 when the mass of the halous acid species (A) is taken as 100.
[2] The liquid composition according to item 1, wherein the content of the halous acid species (A) is 3.0 to 20.0% by mass.
[3] The liquid composition according to item 1 or 2, wherein the mass ratio between the halous acid species (A) and the radical generation catalyst (B) is 1:3 to 3:1.
[4] The liquid composition according to any one of items 1 to 3, wherein the radical generation catalyst (B) is an ammonium salt represented by the following chemical formula (2): 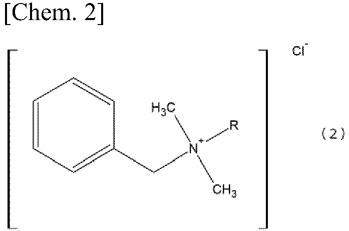 [wherein in the chemical formula (2), R is an alkyl group, and optionally contains an ether bond, a ketone (carbonyl group), an ester bond, or an amide bond, or an aromatic ring].
[5] The liquid composition according to item 4, wherein the ammonium salt represented by the chemical formula (2) is benzethonium chloride and/or benzalkonium chloride.
[6] The liquid composition according to any one of items 1 to 3, wherein the radical generation catalyst (B) is at least one selected from the group consisting of paraquat, ammonium chloride, dequalinium chloride, cetylpyridinium chloride, and didecyldimethylammonium chloride.
[7] The liquid composition according to any one of items 1 to 6, wherein the halous acid of the halous acid species (A) is chlorous acid; the halite ion is a chlorite ion; and the halite salt is a chlorite salt.

### [ADVANTAGEOUS EFFECTS OF INVENTION]

According to the present invention, a liquid composition comprising a radical generation source and a radical generation catalyst at high concentration, the liquid composition having excellent long-term stability, low combustibility, and excellent safety, can be provided.

### DESCRIPTION OF EMBODIMENTS

Embodiments of the present invention are described below in more detail.

In one embodiment, the present invention provides a liquid composition comprising at least:
a halous acid species (A);
a radical generation catalyst (B); and
a buffering agent (C);
the liquid composition satisfying the following conditions (I) to (IV):
   (I) the halous acid species (A) is at least one selected from the group consisting of a halous acid, a halite ion, and a halite salt, and is contained in the liquid composition at 0.3 to 20.0% by mass;
   (II) the radical generation catalyst (B) is an ammonium salt represented by the following chemical formula (1): 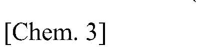 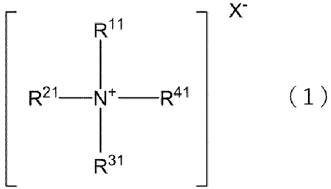 [wherein in the chemical formula (1),
      each of R¹¹, R²¹, R³¹, and R⁴¹ is a hydrogen atom or an alkyl group, and optionally contains an ether bond, a ketone (carbonyl group), an ester bond, or an amide bond, or an aromatic ring; and R¹¹, R²¹, R³¹, and R⁴¹ are the same as or different from each other; or
      two or more of R¹¹, R²¹, R³¹, and R⁴¹ are optionally joined together to form a cyclic structure together with N⁺ to which they are bonded; and the cyclic structure is either saturated or unsaturated, is either an aromatic ring or a non-aromatic ring, and optionally has one or more substituents; and
      X⁻ is an anion (excluding a peroxodisulfate ion)];
      and is contained in the liquid composition at 0.3 to 20.0% by mass;
   (III) the total concentration of the halous acid species (A) and the radical generation catalyst (B) contained in the liquid composition is 1.3 to 30.0% by mass; and
   (IV) the buffering agent (C) contained in the liquid composition contains phosphate ions; the concentration of phosphate ions contained in the liquid composition is 0.01 to 1.50% by mass; and the mass ratio of phosphate ions is not less than 0.33 when the mass of the halous acid species (A) is taken as 100.

Each element constituting the present invention is described below.

### <Halous acid species (A)>

The halous acid species (A) used in the present invention acts as a radical generation source, and may contain at least one selected from the group consisting of a halous acid, a halite ion, and a halite salt. From the viewpoint of mildness of reactivity with the radical generation catalyst and simplicity of control of the reaction, the halous acid species (A) is preferably one selected from the group consisting of chlorous acid, a chlorite ion, and a chlorite salt. In one embodiment, the halous acid species (A) may be sodium chlorite.

In the liquid composition of the present invention, the content of the halous acid species (A) may be 0.3 to 20.0% by mass (for example, the condition (I)) when the amount of the liquid composition is taken as 100% by mass. The content may be, for example, 0.5 to 20.0% by mass, 1.0 to 15.0% by mass, 3.0 to 20.0% by mass, 3.0 to 15.0% by mass, or 4.0 to 12.0% by mass.

### <Radical Generation Catalyst (B)>

The radical generation catalyst (B) used in the present invention has an action of catalyzing radical generation from the radical generation source used in the present invention. A single radical generation catalyst (B) may be used, or two or more radical generation catalysts (B) may be used in combination.

The radical generation catalyst (B) used in the present invention is preferably ammonium having properties as a Lewis acid, or a salt thereof. Such ammonium may be, for example, quaternary ammonium, or may be tertiary, secondary, or primary ammonium. The ammonium is preferably quaternary ammonium.

Examples of the radical generation catalyst (B) used in the present invention include cationic surfactants, especially preferably quaternary ammonium-type cationic surfactants. Examples of the quaternary ammonium-type cationic surfactants include benzalkonium chloride, benzethonium chloride, cetylpyridinium chloride, hexadecyltrimethylammonium bromide, dequalinium chloride, edrophonium, didecyldimethylammonium chloride, tetramethylammonium chloride, tetrabutylammonium chloride, benzyltriethylammonium chloride, oxitropium, carbachol, glycopyrronium, safranine, sinapine, tetraethylammonium bromide, suxamethonium, sphingomyelin, denatonium, trigonelline, neostigmine, paraquat, pyridostigmine, phellodendrine, pralidoxime methiodide, betaine, betanin, bethanechol, betalain, lecithin, and cholines (choline chlorides such as benzoylcholine chloride and lauroylcholine chloride hydrate, phosphocholine, acetylcholine, choline, dipalmitoylphosphatidylcholine, and choline bitartrate). These may be used individually, or two or more of these may be used in combination.

The radical generation catalyst (B) used in the present invention is not limited to the quaternary ammonium-type cationic surfactants described above.

The radical generation catalyst (B) used in the present invention may be, for example, an ammonium salt represented by the following chemical formula (1).

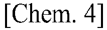

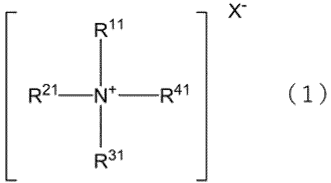

In the chemical formula (1),
each of R¹¹, R²¹, R³¹, and R⁴¹ is a hydrogen atom or an alkyl group, and optionally contains an ether bond, a ketone (carbonyl group), an ester bond, or an amide bond, or an aromatic ring; and R¹¹, R²¹, R³¹, and R⁴¹ are the same as or different from each other; or
two or more of R¹¹, R²¹, R³¹, and R⁴¹ are optionally joined together to form a cyclic structure together with N⁺ to which they are bonded; and the cyclic structure is either saturated or unsaturated, is either an aromatic ring or a non-aromatic ring, and optionally has one or more substituents; and
X⁻ is an anion (excluding a peroxodisulfate ion).

Specific examples of the ammonium salt represented by the chemical formula (1) include benzethonium chloride, benzalkonium chloride, hexadecyltrimethylammonium chloride, tetramethylammonium chloride, ammonium chloride, and tetrabutylammonium chloride. The ammonium salt may be at least one selected from the group consisting of these. In one aspect, the radical generation catalyst (B) may be at least one selected from the group consisting of paraquat, ammonium chloride, dequalinium chloride, cetylpyridinium chloride, and didecyldimethylammonium chloride. Since paraquat, ammonium chloride, dequalinium chloride, cetylpyridinium chloride, and didecyldimethylammonium chloride are quaternary ammonium salts, they function as radical generation catalysts, and exhibit a microbial reduction effect when they are used in combination with a halite salt. Furthermore, since these have a microbial reduction effect even when they are used individually, a synergistic effect can also be expected.

The ammonium salt represented by the chemical formula (1) may be, for example, an ammonium salt represented by the following chemical formula (1a).

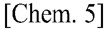

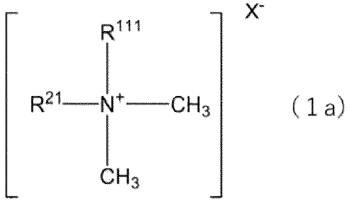

In the chemical formula (1a), R¹¹¹ is an alkyl group having 5 to 40 carbon atoms, and may contain an ether bond, a ketone (carbonyl group), an ester bond, or an amide bond, or an aromatic ring, and R²¹ and X⁻ are the same as in the chemical formula (1).

In the chemical formula (1a), R²¹ is preferably a methyl group or a benzyl group. In the benzyl group, a hydrogen atom(s) on the benzene ring may or may not be substituted with one or more optional substituents, and examples of each of the optional substituents include an alkyl group, an unsaturated aliphatic hydrocarbon group, an aryl group, a heteroaryl group, a halogen, a hydroxy group (-OH), a mercapto group (-SH), and an alkylthio group (-SR, wherein R is an alkyl group).

The ammonium salt represented by the chemical formula (1) is preferably an ammonium salt represented by the following chemical formula (2).

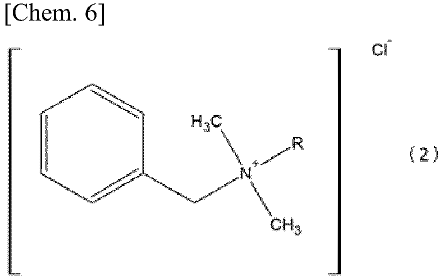

In the chemical formula (2), R is an alkyl group, and may contain an ether bond, a ketone (carbonyl group), an ester bond, or an amide bond, or an aromatic ring. The ammonium salt represented by the chemical formula (2) is preferably benzethonium chloride and/or benzalkonium chloride.

Benzethonium chloride (BzN⁺Cl⁻) can be represented, for example, by the following chemical formula (3). In the formula (3), Me is a methyl group, and ^{t}Bu is a tertiary butyl group. Further, benzalkonium chloride can be represented, for example, by the chemical formula (2) wherein R is an alkyl group having 8 to 18 carbon atoms.

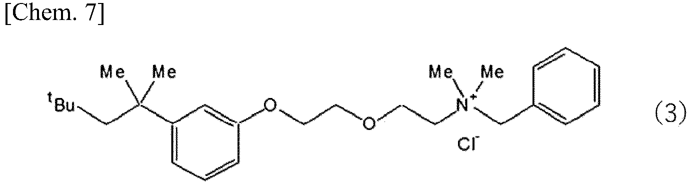

In the chemical formulae (1) and (1a), X⁻ is an anion, and is not particularly limited. Further, X⁻ is not limited to an anion having a monovalent charge, and may also be an anion having any valence such as a valence of 2 or 3. In cases where the anion has a polyvalent charge such as a divalent or trivalent charge, the number of molecules of the ammonium (monovalent) in the chemical formulae (1) and (1a) is, for example, the number of molecules of the anion × the valence of the anion (for example, in cases where the anion is divalent, the number of molecules of the ammonium (monovalent) is twice the number of molecules of the anion). Examples of X⁻ include halide ions (fluoride ions, chloride ions, bromide ions, iodide ions), acetate ions, nitrate ions, and sulfate ions.

Further, the ammonium salt as the radical generation catalyst (B) used in the present invention may contain a plurality of ammonium structures (N⁺) in one molecule. Furthermore, the ammonium salt as the radical generation catalyst (B) used in the present invention may be one in which a plurality of molecules are associated by π-electron interaction to form a dimer, a trimer, or the like.

Further, in cases where isomers such as tautomers or stereoisomers (for example, geometric isomers, conformational isomers, and optical isomers) are present in the radical generation catalyst (B) used in the present invention (for example, an organic ammonium salt), any of the isomers can be used in the present invention unless otherwise specified.

Further, in cases where the radical generation catalyst (B) used in the present invention (for example, an organic ammonium salt) is capable of forming a salt, the salt may be an acid addition salt, or may be a base addition salt. Furthermore, the acid forming the acid addition salt may be either an inorganic acid or an organic acid, and the base forming the base addition salt may be either an inorganic base or an organic base. Examples of the inorganic acid include, but are not particularly limited to, sulfuric acid, phosphoric acid, hydrofluoric acid, hydrochloric acid, hydrobromic acid, hydroiodic acid, hypofluorous acid, hypochlorous acid, hypobromous acid, hypoiodous acid, fluorous acid, chlorous acid, bromous acid, iodous acid, fluoric acid, chloric acid, bromic acid, iodic acid, perfluoric acid, perchloric acid, perbromic acid, and periodic acid. The organic acid is also not particularly limited, and examples thereof include p-toluenesulfonic acid, methanesulfonic acid, oxalic acid, p-bromobenzenesulfonic acid, carbonic acid, succinic acid, citric acid, benzoic acid, and acetic acid. Examples of the inorganic base include, but are not particularly limited to, ammonium hydroxide, alkali metal hydroxides, alkaline earth metal hydroxides, carbonates, and hydrogen carbonates. More specific examples of the inorganic base include sodium hydroxide, potassium hydroxide, potassium carbonate, sodium carbonate, sodium hydrogen carbonate, potassium hydrogen carbonate, calcium hydroxide, and calcium carbonate. The organic base is also not particularly limited, and examples thereof include ethanolamine, triethylamine, and tris(hydroxymethyl)aminomethane. The methods of producing these salts are also not particularly limited. The salts can be produced, for example, by appropriately adding the above-described acid or base to the compound by a known method.

Further, in the radical generation catalyst (B) used in the present invention, each chain substituent (for example, a hydrocarbon group such as an alkyl group or an unsaturated aliphatic hydrocarbon group), unless otherwise specified, may be linear or branched, and the number of carbon atoms thereof is not particularly limited. The number of carbon atoms is preferably 1 to 40, 1 to 32, 1 to 24, 1 to 18, 1 to 12, 1 to 6, or 1 to 2 (in the case of an unsaturated hydrocarbon group, not less than 2) in the order of preference. Further, in the present invention, the number of ring members (the number of atoms constituting the ring) of a cyclic group (for example, an aryl group or a heteroaryl group) is not particularly limited. The number of ring members is preferably 5 to 32, 5 to 24, 6 to 18, 6 to 12, or 6 to 10 in the order of preference. Further, in cases where isomers are present in a substituent or the like, any isomers may be used unless otherwise specified. For example, when a "naphthyl group" is simply mentioned, it may be either a 1-naphthyl group or a 2-naphthyl group.

The radical generation catalyst (B) used in the present invention may be contained in the liquid composition at 0.3 to 20.0% by mass (for example, the condition (II)) when the amount of the liquid composition is taken as 100% by mass. The content is preferably 1.0 to 15.0% by mass, more preferably 2.0 to 10.0% by mass.

In the present invention, the total concentration of the halous acid species (A) and the radical generation catalyst (B) contained in the liquid composition may be 1.3 to 30.0% by mass (for example, the condition (III)) when the amount of the liquid composition is taken as 100% by mass. The total concentration may also be, for example, 2.0 to 25.0% by mass, 3.0 to 25.0% by mass, or 5.0 to 20.0% by mass.

In the liquid composition of the present invention, the concentration ratio between the halous acid species (A) and the radical generation catalyst (B) (halous acid species (A) / radical generation catalyst (B)) is not particularly limited, and may be appropriately set.

Further, in one aspect, the mass ratio between the halous acid species (A) and the radical generation catalyst (B) contained in the liquid composition is preferably 1:3 to 3:1, or may be, for example, 1:2 to 2:1, or about 1:1. In the present description, a numerical value accompanied by "about" may be construed as including a range of ±20%, preferably a range of ±10%, more preferably a range of ±5% of the numerical value.

In the present invention, a buffering agent (C) is used in the liquid composition. The buffering agent (C) has a function of maintaining the pH of the liquid composition within a desired range. In the present invention, for example, a compound containing a carbonate ion, a hydrogencarbonate ion, a borate ion, an ammonium ion, a hydrogen phosphate ion, or a phosphate ion; 3-cyclohexylaminopropanesulfonic acid (CAPS), 3-(cyclohexylamino)-2-hydroxypropane-1-sulfonic acid (CAPSO); or 2-cyclohexylaminoethanesulfonic acid (CHES) may be used. In one aspect, the buffering agent (C) used in the present invention is preferably a compound containing a phosphate ion. The phosphate ion herein may include a hydrogen phosphate ion and a dihydrogen phosphate ion generated by equilibration of phosphate. For example, potassium dihydrogen phosphate, sodium dihydrogen phosphate, dipotassium hydrogen phosphate, disodium hydrogen phosphate, tripotassium phosphate, trisodium phosphate, and the like may be used. These may be used individually, or two or more of these may be used in combination.

In the present invention, the concentration of phosphate ions contained in the liquid composition may be 0.01 to 1.50% by mass (for example, the condition (IV)) when the amount of the liquid composition is taken as 100% by mass. The concentration is preferably 0.05 to 1.50% by mass, more preferably 0.05 to 1.34% by mass. In the present invention, the concentration of phosphate ions contained in the liquid composition in terms of the mass ratio of phosphate ions may be not less than 0.33 (for example, the condition (IV)) when the mass of the halous acid species (A) is taken as 100. The concentration in terms of the mass ratio may be, for example, 0.5, 0.6, 0.7, 0.8, 0.9, 1.0, 1.1, 1.2, 1.3, 1.4, or 1.5 or more.

In one aspect, the liquid composition of the present invention contains water as a solvent for dissolving or dispersing the halous acid species (A), the radical generation catalyst (B), and the buffering agent (C). Examples of the water include purified water, ion-exchanged water, distilled water, filtered water, and sterilized water.

The liquid composition of the present invention preferably has a neutral or alkaline property. In cases where the liquid composition is acidic, the radical generation source and the radical generation catalyst react rapidly to cause generation of radicals, so that long-term stable generation of radicals cannot be easily achieved. In the present invention, aqueous radicals (active species) generated in the liquid composition maintain their state under a neutral or alkaline environment. When bacteria, viruses, and the like as a target of reaction are present, aqueous radicals present in the liquid composition act on the target, resulting in disappearance of these radicals. However, new radicals are generated from the liquid composition. As a result, radicals can be generated when needed. In the liquid composition of the present invention, the pH immediately after the production is preferably 7 to 13, or may be, for example, 8 to 13, 9 to 13, or 10 to 12.

The liquid composition of the present invention can be produced by dissolving the halous acid species (A), the radical generation catalyst (B), and the buffering agent (C) in a solvent and, when necessary, adjusting the pH.

The liquid composition provided by the present invention is chemically stable over a long period, and exhibits an excellent effect by which, for example, pH changes and decomposition of the halous acid species can be reduced even under conditions of storage at 40°C for 6 months. The liquid composition provided by the present invention also has low combustibility and high safety. Furthermore, the liquid composition provided by the present invention exhibits a sufficient microbial reduction effect even in cases where the halous acid species contained in the liquid composition is diluted to a concentration that is normally used (for example, about 160 ppm by mass of the halous acid species). The liquid composition provided by the present invention is used in a state where the halous acid species contained in the liquid composition, which acts as a radical generation source, is diluted to an extent at which a microbial reduction effect is exerted. Therefore, the liquid composition can be transported in a highly concentrated state until it is transported to a desired place.

The method and the dilution factor for diluting the liquid composition provided by the present invention for use as a disinfecting agent are not particularly limited. For example, the halous acid species is preferably mixed, in the solution immediately before use, at preferably 0.01 to 1500 ppm by mass, more preferably 0.05 to 1000 ppm by mass, still more preferably 0.1 to 250 ppm by mass. The solvent used for the dilution may be water, or may be an aqueous solution in which the buffering agent (C) is dissolved.

### EXAMPLES

The present invention is described below by way of examples in more detail. However, the present invention is not limited to the following examples.

### [Experiment 1]

Sodium chlorite as the halous acid species (A), benzethonium chloride or benzalkonium chloride as the radical generation catalyst (B), and trisodium phosphate dodecahydrate as the buffering agent (C), were dissolved in pure water to achieve the concentrations shown in tables (Tables 1-1 to 1-3 and Table 2), to prepare Test Samples 1 to 135. It should be noted that the tables also show the phosphate ion concentration (% by mass in the liquid composition) in each case where trisodium phosphate dodecahydrate was dissolved.

### <Test Sample Compositions>

**[Table 1-1]**

| Test Sample No. | Concentration in liquid com | | position [mass%] | | | Mass ratio of phosphate ions to (A) 100 mass% | Test results | | |
|---|---|---|---|---|---|---|---|---|---|
| | (A) | (B) | Sum of (A) + (B) | (C) | | | | | |
| | Sodium chlorite | Benzethonium chloride | | Trisodium phosphate dodecahydrate (*) | ((*) Mass% of "phosphate ions") | | Stability Test (1) | Stability Test (2) | Combustibility test |
| 1 | 20 | 0 | 20 | 0.00 | 0.00 | 0.00 | ++ | ++ | + |
| 2 | 0 | 20 | 20 | 0.00 | 0.00 | (-) | ++ | N/A | + |
| 3 | 0 | 0 | 0 | 6.00 | 1.50 | (-) | ++ | N/A | + |
| 4 | 20 | 20 | 40 | 0.00 | 0.00 | 0.00 | - | - | - |
| 5 | | | | 1.34 | 0.33 | 1.68 | - | - | - |
| 6 | 15 | 15 | 30 | 0.00 | 0.00 | 0.00 | - | - | + |
| 7 | | | | 0.03 | 0.01 | 0.04 | - | - | + |
| 8 | | | | 0.13 | 0.03 | 0.22 | + | ++ | + |
| 9 | | | | 0.27 | 0.07 | 0.45 | ++ | ++ | + |
| 10 | | | | 2.68 | 0.67 | 4.48 | +++ | +++ | + |
| 11 | | | | 5.35 | 1.34 | 8.90 | +++ | +++ | + |
| 12 | | | | 6.00 | 1.50 | 10.00 | ++ | +++ | + |
| 13 | 10 | 10 | 20 | 0.00 | 0.00 | 0.00 | - | - | + |
| 14 | | | | 0.03 | 0.01 | 0.07 | - | - | + |
| 15 | | | | 0.13 | 0.03 | 0.33 | ++ | ++ | + |
| 16 | | | | 0.27 | 0.07 | 0.67 | ++ | +++ | + |
| 17 | | | | 2.68 | 0.67 | 6.69 | +++ | +++ | + |
| 18 | | | | 5.35 | 1.34 | 13.38 | +++ | +++ | + |
| 19 | | | | 6.00 | 1.50 | 15.00 | ++ | +++ | + |
| 20 | 5 | 5 | 10 | 0.00 | 0.00 | 0.00 | - | - | + |
| 21 | | | | 0.03 | 0.01 | 0.13 | + | + | + |
| 22 | | | | 0.13 | 0.03 | 0.67 | ++ | +++ | + |
| 23 | | | | 0.27 | 0.07 | 1.34 | +++ | +++ | + |
| 24 | | | | 2.68 | 0.67 | 13.38 | +++ | +++ | + |
| 25 | | | | 5.35 | 1.34 | 26.76 | +++ | +++ | + |
| 26 | | | | 6.00 | 1.50 | 30.00 | ++ | +++ | + |
| 27 | 1 | 1 | 2 | 0.00 | 0.00 | 0.00 | - | - | + |
| 28 | | | | 0.03 | 0.01 | 0.67 | ++ | +++ | + |
| 29 | | | | 0.13 | 0.03 | 3.35 | +++ | +++ | + |
| 30 | | | | 0.27 | 0.07 | 6.69 | +++ | +++ | + |
| 31 | | | | 2.68 | 0.67 | 66.90 | +++ | +++ | + |
| 32 | | | | 5.35 | 1.34 | 133.80 | +++ | +++ | + |
| 33 | | | | 6.00 | 1.50 | 150.00 | ++ | +++ | + |

**[Table 1-2]**

| Test Sample No. | Concentration in liquid composition [mass%] | | | | | Mass ratio of phosphate ions to (A) 100 mass% | Test results | | |
|---|---|---|---|---|---|---|---|---|---|
| | (A) | (B) | Sum of (A) + (B) | (C) | | | | | |
| | Sodium chlorite | Benzethonium chloride | | Trisodium phosphate dodecahydrate (*) | ((*) Mass% of "phosphate ions") | | Stability Test (1) | Stability Test (2) | Combustibility test |
| 34 | 30 | 10 | 40 | 0.00 | 0.00 | 0.00 | - | - | - |
| 35 | | | | 1.34 | 0.33 | 1.07 | - | - | - |
| 36 | 20 | 6.7 | 26.7 | 0.00 | 0.00 | 0.00 | - | - | + |
| 37 | | | | 0.03 | 0.01 | 0.03 | - | - | + |
| 38 | | | | 0.13 | 0.03 | 0.17 | + | + | + |
| 39 | | | | 0.27 | 0.07 | 0.33 | ++ | ++ | + |
| 40 | | | | 1.34 | 0.33 | 1.67 | +++ | +++ | + |
| 41 | | | | 2.68 | 0.67 | 3.35 | +++ | +++ | + |
| 42 | | | | 5.35 | 1.34 | 6.69 | +++ | +++ | + |
| 43 | | | | 6.00 | 1.50 | 7.50 | ++ | +++ | + |
| 44 | 15 | 5 | 20 | 0.00 | 0.00 | 0.00 | - | - | + |
| 45 | | | | 0.03 | 0.01 | 0.04 | - | - | + |
| 46 | | | | 0.13 | 0.03 | 0.22 | + | ++ | + |
| 47 | | | | 0.27 | 0.07 | 0.45 | ++ | ++ | + |
| 48 | | | | 2.68 | 0.67 | 4.48 | +++ | +++ | + |
| 49 | | | | 5.35 | 1.34 | 8.90 | +++ | +++ | + |
| 50 | | | | 6.00 | 1.50 | 10.00 | ++ | +++ | + |
| 51 | 10 | 3.3 | 13.3 | 0.00 | 0.00 | 0.00 | - | - | + |
| 52 | | | | 0.03 | 0.01 | 0.07 | - | - | + |
| 53 | | | | 0.13 | 0.03 | 0.33 | ++ | ++ | + |
| 54 | | | | 0.27 | 0.07 | 0.67 | ++ | +++ | + |
| 55 | | | | 2.68 | 0.67 | 6.69 | +++ | +++ | + |
| 56 | | | | 5.35 | 1.34 | 13.38 | +++ | +++ | + |
| 57 | | | | 6.00 | 1.50 | 15.00 | ++ | +++ | + |
| 58 | 5 | 1.7 | 6.7 | 0.00 | 0.00 | 0.00 | - | - | + |
| 59 | | | | 0.03 | 0.01 | 0.13 | + | + | + |
| 60 | | | | 0.13 | 0.03 | 0.67 | ++ | +++ | + |
| 61 | | | | 0.27 | 0.07 | 1.34 | +++ | +++ | + |
| 62 | | | | 2.68 | 0.67 | 13.38 | +++ | +++ | + |
| 63 | | | | 5.35 | 1.34 | 26.76 | +++ | +++ | + |
| 64 | | | | 6.00 | 1.50 | 30.00 | ++ | +++ | + |
| 65 | 1 | 0.3 | 1.3 | 0.00 | 0.00 | 0.00 | - | - | + |
| 66 | | | | 0.03 | 0.01 | 0.67 | ++ | +++ | + |
| 67 | | | | 0.27 | 0.07 | 6.69 | +++ | +++ | + |
| 68 | | | | 2.68 | 0.67 | 66.90 | +++ | +++ | + |
| 69 | | | | 5.35 | 1.34 | 133.80 | +++ | +++ | + |
| 70 | | | | 6.00 | 1.50 | 150.00 | ++ | +++ | + |

**[Table 1-3]**

| Test Sample No. | Concentration in liquid com | | position [mass%] | | | Mass ratio of phosphate ions to (A) 100 mass% | Test results | | |
|---|---|---|---|---|---|---|---|---|---|
| | (A) | (B) | Sum of (A) + (B) | (C) | | | | | |
| | Sodium chlorite | Benzethonium chloride | | Trisodium phosphate dodecahydrate (*) | ((*) Mass% of "phosphate ions") | | Stability Test (1) | Stability Test (2) | Combustibility test |
| 71 | 10 | 30 | 40 | 0.00 | 0.00 | 0.00 | - | - | + |
| 72 | | | | 1.34 | 0.33 | 3.35 | - | - | + |
| 73 | 6.7 | 20 | 26.7 | 0.00 | 0.00 | 0.00 | - | - | + |
| 74 | | | | 0.03 | 0.01 | 0.10 | + | + | + |
| 75 | | | | 0.13 | 0.03 | 0.50 | ++ | ++ | + |
| 76 | | | | 0.27 | 0.07 | 1.00 | ++ | +++ | + |
| 77 | | | | 2.68 | 0.67 | 10.04 | +++ | +++ | + |
| 78 | | | | 5.35 | 1.34 | 20.07 | +++ | +++ | + |
| 79 | | | | 6.00 | 1.50 | 22.50 | ++ | +++ | + |
| 80 | 5 | 15 | 20 | 0.00 | 0.00 | 0.00 | - | - | + |
| 81 | | | | 0.03 | 0.01 | 0.13 | + | + | + |
| 82 | | | | 0.13 | 0.03 | 0.67 | ++ | +++ | + |
| 83 | | | | 0.27 | 0.07 | 1.34 | +++ | +++ | + |
| 84 | | | | 2.68 | 0.67 | 13.38 | +++ | +++ | + |
| 85 | | | | 5.35 | 1.34 | 26.76 | +++ | +++ | + |
| 86 | | | | 6.00 | 1.50 | 30.00 | ++ | +++ | + |
| 87 | 3.3 | 10 | 13.3 | 0.00 | 0.00 | 0.00 | - | - | + |
| 88 | | | | 0.03 | 0.01 | 0.20 | + | + | + |
| 89 | | | | 0.13 | 0.03 | 1.00 | ++ | +++ | + |
| 90 | | | | 0.27 | 0.07 | 2.01 | +++ | +++ | + |
| 91 | | | | 2.68 | 0.67 | 20.07 | +++ | +++ | + |
| 92 | | | | 5.35 | 1.34 | 40.14 | +++ | +++ | + |
| 93 | | | | 6.00 | 1.50 | 45.00 | ++ | +++ | + |
| 94 | 1.7 | 5 | 6.7 | 0.00 | 0.00 | 0.00 | - | - | + |
| 95 | | | | 0.03 | 0.01 | 0.40 | ++ | ++ | + |
| 96 | | | | 0.13 | 0.03 | 2.01 | +++ | +++ | + |
| 97 | | | | 0.27 | 0.07 | 4.01 | +++ | +++ | + |
| 98 | | | | 2.68 | 0.67 | 40.14 | +++ | +++ | + |
| 99 | | | | 5.35 | 1.34 | 80.28 | +++ | +++ | + |
| 100 | | | | 6.00 | 1.50 | 90.00 | ++ | +++ | + |
| 101 | 0.3 | 1 | 1.3 | 0.00 | 0.00 | 0.00 | - | - | + |
| 102 | | | | 0.03 | 0.01 | 2.01 | +++ | +++ | + |
| 103 | | | | 0.13 | 0.03 | 10.04 | +++ | +++ | + |
| 104 | | | | 0.27 | 0.07 | 20.07 | +++ | +++ | + |
| 105 | | | | 2.68 | 0.67 | 200.70 | +++ | +++ | + |
| 106 | | | | 5.35 | 1.34 | 401.40 | +++ | +++ | + |
| 107 | | | | 6.00 | 1.50 | 450.00 | ++ | +++ | + |

**[Table 2]**

| Test Sample No. | Concentration in liquid composition [mass%] | | | | | Mass ratio of phosphate ions to (A) 100 mass% | Test results | | |
|---|---|---|---|---|---|---|---|---|---|
| | (A) | (B) | Sum of (A) + (B) | (C) | | | | | |
| | Sodium chlorite | Benzalkonium chloride | | Trisodium phosphate dodecahydrate (*) | ((*) Mass% of "phosphate ions") | | Stability Test (1) | Stability Test (2) | Combustibility test |
| 108 | 0 | 20 | 20 | 0.00 | 0.00 | (-) | ++ | N/A | + |
| 109 | 20 | 20 | 40 | 0.00 | 0.00 | 0.00 | - | - | - |
| 110 | | | | 1.34 | 0.33 | 1.68 | - | - | - |
| 111 | 15 | 15 | 30 | 0.00 | 0.00 | 0.00 | - | - | + |
| 112 | | | | 0.03 | 0.01 | 0.04 | - | - | + |
| 113 | | | | 0.13 | 0.03 | 0.22 | + | ++ | + |
| 114 | | | | 0.27 | 0.07 | 0.45 | ++ | +++ | + |
| 115 | | | | 2.68 | 0.67 | 4.48 | +++ | +++ | + |
| 116 | | | | 5.35 | 1.34 | 8.90 | +++ | +++ | + |
| 117 | | | | 6.00 | 1.50 | 10.00 | ++ | +++ | + |
| 118 | 30 | 10 | 40 | 0.00 | 0.00 | 0.00 | - | - | - |
| 119 | | | | 1.34 | 0.33 | 1.07 | - | - | - |
| 120 | 20 | 6.7 | 26.7 | 0.00 | 0.00 | 0.00 | - | - | + |
| 121 | | | | 0.03 | 0.01 | 0.03 | - | - | + |
| 122 | | | | 0.13 | 0.03 | 0.17 | + | + | + |
| 123 | | | | 0.27 | 0.07 | 0.33 | ++ | ++ | + |
| 124 | | | | 1.34 | 0.33 | 1.67 | +++ | +++ | + |
| 125 | | | | 2.68 | 0.67 | 3.35 | +++ | +++ | + |
| 126 | | | | 5.35 | 1.34 | 6.69 | +++ | +++ | + |
| 127 | | | | 6.00 | 1.50 | 7.50 | ++ | +++ | + |
| 128 | 10 | 30 | 40 | 0.00 | 0.00 | 0.00 | - | - | + |
| 129 | | | | 1.34 | 0.33 | 3.35 | - | - | + |
| 130 | 6.7 | 20 | 26.7 | 0.00 | 0.00 | 0.00 | - | - | + |
| 131 | | | | 0.03 | 0.01 | 0.10 | + | + | + |
| 132 | | | | 0.13 | 0.03 | 0.50 | ++ | ++ | + |
| 132 | | | | 0.27 | 0.07 | 1.00 | ++ | +++ | + |
| 133 | | | | 2.68 | 0.67 | 10.04 | +++ | +++ | + |
| 134 | | | | 5.35 | 1.34 | 20.07 | +++ | +++ | + |
| 135 | | | | 6.00 | 1.50 | 22.50 | ++ | +++ | + |

### 2. Test Results

The respective test results in the above tables are presented in accordance with the following criteria.

### <Stability Test (1)>

Each test sample (100 mL) was dispensed into a 100-mL polyethylene bottle, and stored for 6 months in a constant-temperature bath set at 40°C. Thereafter, the change in the pH was confirmed by the glass electrode method. A sample with a pH change of not less than 3.0 was rated as "-"; a sample with a pH change of not less than 2.0 and less than 3.0 was rated as "+"; a sample with a pH change of not less than 1.0 and less than 2.0 was rated as "++"; and a sample with a pH change of less than 1.0 was rated as "+++".

### <Stability Test (2)>

Each test sample (100 mL) was dispensed into a 100-mL polyethylene bottle, and stored for 6 months in a constant-temperature bath set at 40°C. Thereafter, the chlorite ion concentration and the chlorate ion concentration were each measured by anion chromatography (see Table 3 below). The chlorate ion concentration was determined as a relative value to the chlorite ion concentration, which was taken as 100. A sample with a value of not less than 20 was rated as "-"; a sample with a value of not less than 10 and less than 20 was rated as "+"; a sample with a value of not less than 5 and less than 10 was rated as "++"; and a sample with a value of less than 5 was rated as "+++". "N/A" indicates a sample for which the test was not carried out because it is a sample containing no chlorite ion.

### Conditions for anion chromatography

**[Table 3]**

| | |
|---|---|
| Column | Thermo Dionex Ion Pac AS12A 4 × 200 mm |
| Eluent | 2.7 mmol/L Na₂CO₃ and 0.3 mmol/L NaHCO₃ for Dionex Ion Pac AS12A |
| Temperature | 35°C |
| Flow rate | 1.3 mL/min |
| Detection | Electrical conductivity |

### <Combustibility Test (Safety)>

Wood flour (sapwood of Japanese cedar) having a particle size that allows passing through a standard wire sieve with an opening of 500 µm but that does not allow passing through a standard wire sieve with an opening of 250 µm was dried at 105°C for 4 hours, and stored at 20±5°C for not less than 24 hours in a desiccator containing drying silica gel. After placing 15 g of the wood flour in a conical cup, the cup was inverted on a flat-bottom evaporating dish to form a conical deposit. This conical deposit was left to stand for 1 hour at a test site (temperature: 20±5°C; humidity: 50±10 RH%). Using a syringe, 15 g of the test sample was uniformly poured from above the wood flour. As a combustibility test, a test used for determination of Class 6 hazardous materials (oxidizing liquids) under the Fire Service Act was carried out. Specifically, an ignition tool prepared by forming a nichrome wire into a ring shape and heating it to about 1000°C by energization was used. This ignition tool was brought into contact, from above, with the base of the conical deposit prepared as described above, and was kept in contact for up to 10 seconds until complete ignition occurred with flaming, or until it became clear that no ignition occurred at all. In this test, the time to flame extinction was measured to evaluate danger. When the time was not less than 30 seconds, the sample was rated as "-", and when the time was less than 30 seconds, the sample was rated as "+".

The initial pHs immediately after the production of Test Sample Nos. 4, 5, 6, 8, 10, 13, 15, 17, 20, 22, and 24 were 10 to 12 as follows, and were pHs at which radicals could be generated when necessary.

**[Table 4]**

| Test Sample No. | Concentration in liquid composition [mass%] | | | | | Mass ratio of phosphate ions to (A) 100 mass% | Test result Stability Test (1) | Initial pH |
|---|---|---|---|---|---|---|---|---|
| | (A) | (B) | Sum of (A) + (B) | (C) | | | | |
| | Sodium chlorite | Benzethonium chloride | | Trisodium phosphate dodecahydrate (*) | ((*) Mass% of "phosphate ions") | | | |
| 4 | 20 | 20 | 40 | 0.00 | 0.00 | 0.00 | - | 10.8 |
| 5 | | | | 1.34 | 0.33 | 1.68 | - | 11.5 |
| 6 | 15 | 15 | 30 | 0.00 | 0.00 | 0.00 | - | 10.8 |
| 8 | | | | 0.13 | 0.03 | 0.22 | + | 11.2 |
| 10 | | | | 2.68 | 0.67 | 4.48 | +++ | 11.6 |
| 13 | 10 | 10 | 20 | 0.00 | 0.00 | 0.00 | - | 10.7 |
| 15 | | | | 0.13 | 0.03 | 0.33 | ++ | 11.2 |
| 17 | | | | 2.68 | 0.67 | 6.69 | +++ | 11.7 |
| 20 | 5 | 5 | 10 | 0.00 | 0.00 | 0.00 | - | 10.6 |
| 22 | | | | 0.13 | 0.03 | 0.67 | ++ | 11.2 |
| 24 | | | | 2.68 | 0.67 | 13.38 | +++ | 11.7 |

### [Experiment 2]

### <Test for Confirming Microbial Reduction Effect (Functionality)>

Test Sample Nos. 4 to 135 were mixed with Mueller-Hinton II liquid medium to achieve a sodium chlorite concentration of 160 ppm by mass, thereby preparing test samples. In addition, 10 g of meat extract, 10 g of peptone, 3 g of sodium chloride, and 1000 g of distilled water were mixed together, and the resulting mixture was heated to allow dissolution, followed by adjusting the pH to 7.2 to 7.4 and autoclaving at 121°C for 15 minutes, thereby obtaining a normal medium. *Escherichia coli* as a test bacterium was inoculated into the normal medium, and cultured at 32.5°C for 24 hours. Thereafter, the bacterial count was adjusted with physiological saline to 10⁷/mL, thereby obtaining a test bacterial liquid.

The test bacterial liquid was inoculated into each test sample, and culture was performed for 24 hours in a constant-temperature bath set at 32.5°C. Thereafter, the presence or absence of the growth of the test bacterium was visually observed.

In the test samples of Test Sample Nos. 4 to 135, no growth of the test bacterium was observed.

## Claims

1. A liquid composition comprising at least:
a halous acid species (A);
a radical generation catalyst (B); and
a buffering agent (C);
the liquid composition satisfying the following conditions (I) to (IV):
(I) the halous acid species (A) is at least one selected from the group consisting of a halous acid, a halite ion, and a halite salt, and is contained in the liquid composition at 0.3 to 20.0% by mass;
(II) the radical generation catalyst (B) is an ammonium salt represented by the following chemical formula (1): 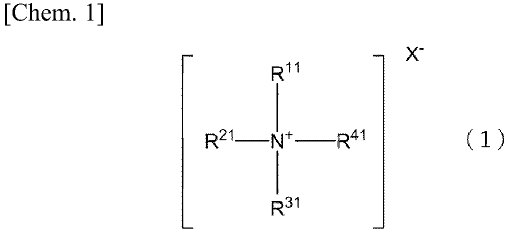 [wherein in the chemical formula (1),
each of R¹¹, R²¹, R³¹, and R⁴¹ is a hydrogen atom or an alkyl group, and optionally contains an ether bond, a ketone (carbonyl group), an ester bond, or an amide bond, or an aromatic ring; and R¹¹, R²¹, R³¹, and R⁴¹ are the same as or different from each other; or
two or more of R¹¹, R²¹, R³¹, and R⁴¹ are optionally joined together to form a cyclic structure together with N⁺ to which they are bonded; and the cyclic structure is either saturated or unsaturated, is either an aromatic ring or a non-aromatic ring, and optionally has one or more substituents; and
X⁻ is an anion (excluding a peroxodisulfate ion)];
and is contained in the liquid composition at 0.3 to 20.0% by mass;
(III) the total concentration of the halous acid species (A) and the radical generation catalyst (B) contained in the liquid composition is 1.3 to 30.0% by mass; and
(IV) the buffering agent (C) contained in the liquid composition contains phosphate ions; the concentration of phosphate ions contained in the liquid composition is 0.01 to 1.50% by mass; and the mass ratio of phosphate ions is not less than 0.33 when the mass of the halous acid species (A) is taken as 100.

2. The liquid composition according to claim 1, wherein the content of the halous acid species (A) is 3.0 to 20.0% by mass.

3. The liquid composition according to claim 1, wherein the mass ratio between the halous acid species (A) and the radical generation catalyst (B) is 1:3 to 3:1.

4. The liquid composition according to claim 1, wherein the radical generation catalyst (B) is an ammonium salt represented by the following chemical formula (2): 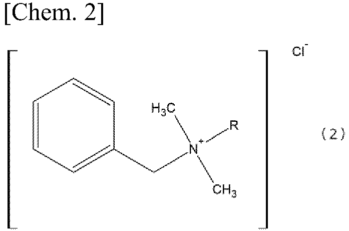 [wherein in the chemical formula (2), R is an alkyl group, and optionally contains an ether bond, a ketone (carbonyl group), an ester bond, or an amide bond, or an aromatic ring].

5. The liquid composition according to claim 4, wherein the ammonium salt represented by the chemical formula (2) is benzethonium chloride and/or benzalkonium chloride.

6. The liquid composition according to claim 1, wherein the radical generation catalyst (B) is at least one selected from the group consisting of paraquat, ammonium chloride, dequalinium chloride, cetylpyridinium chloride, and didecyldimethylammonium chloride.

7. The liquid composition according to claim 1, wherein the halous acid of the halous acid species (A) is chlorous acid; the halite ion is a chlorite ion; and the halite salt is a chlorite salt.
